# EUROPEAN PATENT APPLICATION

(11) **EP 4 697 754 A1**
(43) Date of publication of application: **18.02.2026**
(21) Application number: 25194173.8
(22) Date of filing: 05.08.2025
(51) Int. Cl.: H04R 25/00, A61N 1/372, A61N 1/378, H02J 50/12

(54) **A RECEIVER FOR A BONE-ANCHORED HEARING SYSTEM HAVING MULTIPLE COILS, AND A BONE-ANCHORED HEARING SYSTEM**

(30) Priority: 07.08.2024 EP 24193303
(71) Applicant: Oticon Medical A/S, 2765 Smørum (DK)
(72) Inventor: KOCHER, Johannes, 43632 Askim (SE); JANSSON, Karl-Johan Fredén, 412 96 Göteborg (SE); LIANG, Chen, 43632 Askim (SE); PIERQUIN, Regis, FR-06220 Vallauris (FR)
(74) Representative: Demant

(57) **Abstract**

A receiver for a bone-anchored hearing system (BAHS) is disclosed the receiver comprises a data transfer antenna tuned at a first resonant frequency and configured to receive a transmitted data signal from a transmitter, wherein the data transfer antenna is configured to provide a data signal based on the transmitted data signal; an energy receiver coil tuned at a second resonant frequency and configured to receive a transmitted energy signal from the transmitter, wherein the energy receiver coil is configured to provide an energy signal based on the transmitted energy signal; a receiver circuit configured to receive the energy signal and the data signal, wherein the receiver circuit is configured to convert the data signal to an output signal; and a vibrator transducer configured to receive the output signal and to provide, based on the output signal, a vibration perceivable as sound by the user.

## Description

### TECHNICAL FIELD

The present application generally relates to the field of bone-anchored hearing systems. In particular, the present application generally relates to the field of receivers for bone-anchored hearing systems.

### BACKGROUND

Hearing by bone conduction as a phenomenon, e.g., conduction of sound to the inner ear through the bones of the skull, is known. Bone conduction transducers combine properties such as small size, wide frequency range, and efficient energy transformation; hence, they are widely used in hearing aid applications.

A transcutaneous bone-anchored hearing aid device includes at least the transducer which is implanted beneath the skin layers and fixated onto the skull of a user, and in most cases the transducer is coupled to another housing including at least a receiver coil which is also implanted beneath the skin layers and fixated onto the skull of the user. The receiver coil may receive an external generated communication signal from an external device fixated onto the skin of the user, so as to apply vibrations to the skull which are perceived as indicative of a sound environment around the user.

### SUMMARY

### A receiver for a bone-anchored hearing system:

In an aspect of the present disclosure, embodiments of a receiver for a bone-anchored hearing system (BAHS) are disclosed. The receiver includes a data transfer antenna. The data transfer antenna is tuned at a first resonant frequency and configured to receive a transmitted data signal from a transmitter. The data transfer antenna is configured to provide a data signal based on the transmitted data signal. The receiver includes an energy receiver coil. The energy receiver coil is tuned at a second resonant frequency and configured to receive a transmitted energy signal from the transmitter. The energy receiver coil is configured to provide an energy signal based on the transmitted energy signal. The receiver further includes a receiver circuit. The receiver circuit is configured to receive the energy signal and the data signal. The receiver circuit is configured to convert the data signal to an output signal. The receiver further includes a vibrator transducer. The vibrator transducer is configured to receive the output signal. The vibrator transducer is configured to provide, based on the output signal, a vibration perceivable as sound by the user.

Embodiments of the disclosed receiver include improved transcutaneous electric transfer (TET). The communication between a transmitter and a receiver may be known as a TET link. Typical TET links tend to have bad efficiency losses between 50-70 percent due to the bad permeability of the skin that is between the transmitter antenna and the receiver coil (e.g., loose coupling). This link efficiency is further weakened by the fact that both energy and data is transferred through the link. The data is a wideband signal, decreasing the quality factor (Q-factor) of the link in a compromise to transfer signal data. In certain examples, a 2-3dB improvement, and even up to 6dB, can be achieved using embodiments of the disclosed receiver.

Advantageously, the disclosed receiver addresses the low efficiency of the link by adding an additional coil into the receiver. Having two coils can separate the energy transfer received by the receiver and the data transfer received by the receiver. Specifically, frequency separation between data carrier frequencies and energy carrier frequencies can allow for optimization of signal transfer. Moreover, the disclosed receiver allows for the use of multiple frequency bands with different regulatory allowed energy levels. Additionally, the data transfer antenna and the energy receiver coil are not required to have the exact same resonance frequency in certain embodiments, which allows for the highest power transfer. Further, with the data transfer antenna being separate from the energy receiver coil, the specific electronic components around the respective antenna and coil can be optimized. For example, a high-Q value can be created for each of the resonant circuits.

In certain examples, one coil will be tuned with one Q-factor to transfer the signal data, the second coil will be tuned with a second Q-factor and frequency band to transfer energy more efficient. This can greatly improve transmission efficiency for the receiver.

The receiver can be considered a part of a bone-anchored hearing system (BAHS). The receiver can be implanted into a hearing aid user. For example, the receiver can be transcutaneously implanted into a hearing aid user. The receiver can be subcutaneously implanted into a hearing aid user. In certain example, the receiver can be implanted to be in contact with the skull of a hearing aid user. In certain examples, the receiver can be attached to the skull of a hearing aid user. In one or more example receivers, the receiver is configured to be anchored to a skull of a user.

The receiver can include a data transfer antenna. The data transfer antenna may be a data transfer coil. The data transfer antenna can be a radiofrequency (RF) antenna. The data transfer antenna can be a RF antenna operating at 2.4GHz (e.g., a Bluetooth low energy (BLE) configuration). The data transfer antenna can be configured to receive a transmitted data signal. The data transfer antenna can be configured to receive a transmitted data signal from a transmitter.

The transmitted data signal can be indicative of a sound environment around the user. The transmitted data signal can be indicative of a streaming sound. For example, the transmitted data signal can be indicative of sounds received by one or more microphones. The transmitted data signal can be indicative of control data for the receiver. The transmitted data signal can be indicative of a change in operating parameters of the receiver. For example, the transmitted data signal can be indicative of a change in the first resonant frequency and/or the second resonant frequency.

The data transfer antenna is configured to provide a data signal based on the transmitted data signal. The data signal can be based on the transmitted data signal. The data signal can include information from the transmitted data signal.

The data signal can include a representation of sound. The data signal can be indicative of a sound environment around the user. The data signal can be indicative of a streaming sound.

The data signal can be representative of control data. For example, the data signal can be indicative of control data configured for setting active parameters of the receiver. The data signal can be indicative of control data configured for setting security parameters. The data signal can be indicative of a change of one or more operating parameters of the receiver.

The data transfer antenna can be tuned at a first resonant frequency. The first resonant frequency can be a frequency between 120kHz and 7 MHz. The first resonant frequency can be Bluetooth 2.4GHz frequencies. The first resonant frequency can be between 300-400kHz.

The receiver can further include an energy receiver coil. The energy receiver coil can be configured to receive a transmitted energy signal. The energy receiver coil can be configured to receive a transmitted energy signal from a transmitter. The energy receiver coil can be configured to receive and temporarily store a transmitted energy signal from a transmitter.

The energy receiver coil is configured to provide an energy signal based on the transmitted energy signal. The energy signal can be based on the transmitted energy signal. The energy receiver coil can be optimized by using fragmantises coils.

The energy receiver coil is tuned to a second resonant frequency. The second resonant frequency can be a frequency between 120kHz and 7 MHz.

In one or more example receivers, the first resonant frequency is different from the second resonant frequency. This difference can separate the data and energy signals from each other. Further, this difference could allow for a smaller size of coil intended for energy transfer.

In one or more example receivers, the first resonant frequency is the same as the second resonant frequency. Using two identical coils would double the number of winding turns as compared to using only one of the coils. This can increase the receiver inductance. This can increase the voltage level for the receiver.

In certain examples, the data transfer antenna can be tuned at a Q-factor lower than a Q-factor of the energy receiver coil.

The receiver further includes a receiver circuit. In some examples, the receiver circuit can be a receiver processor. The receiver circuit can be a signal processor. In certain embodiments, the receiver circuit is a processor chip. The receiver circuit is configured to receive the energy signal. The receiver circuit is configured to receive the data signal.

The receiver can power one or more components with the energy signal. For example, the receiver can power one or more amplifiers with the energy signal. The receiver can charge one or more capacitors with the energy signal. The receiver can power the vibrator transducer with the energy signal. If a full processor chip were incorporated into the receiver, the receiver can power the processor chip with the energy signal.

In one or more examples, the receiver can provide the energy signal to an implantable battery (e.g., load the implantable battery). The receiver can provide the energy signal to the implantable battery wirelessly. The receiver can provide the energy signal to the implantable battery via a wired connection.

The receiver circuit can be configured to operate one or more components of the receiver. The receiver circuit can be configured to process the data signal. As an example, the receiver circuit can convert a digital signal to an analog signal. The receiver circuit can perform further functionality as well (e.g., the receiver circuit may be a receiver processor), depending on the receiver circuit. For example, the receiver circuit can be configured to calculate charge for determination of the amount of energy signal to put into and/or pull out of a transducer to achieve the audio signal for transmission to the user. The receiver circuit can perform temperature calculations and compensations based on said temperature calculations. The receiver circuit can perform calibration calculations and/or measurements and can apply calibrations based on the calibration calculations and/or measurements.

The receiver circuit is configured to convert the data signal to an output signal. The output signal can be based on the data signal. The output signal can be based on the transmitted data signal. The output signal can be indicative of sound to be converted into vibrations via the vibrator transducer.

The receiver can further include a vibrator transducer (e.g., vibrator, vibration unit). The vibrator transducer can be electromagnetic. The vibrator transducer can be piezoelectric. The vibrator transducer is configured to receive the output signal. The vibrator transducer is configured to provide, based on the output signal, a vibration perceivable as sound by a user. The vibrator transducer is configured to provide, based on the output signal, a vibration perceivable as sound by a user that is indicative of a sound environment of the user. The vibrator transducer is configured to vibrate, based on the output signal, in a manner perceivable as sound by a user.

In one or more example receivers, the data transfer antenna is configured to transmit a receiver data signal. For example, the data transfer antenna is configured to transmit a receiver data signal to a transmitter of the bone-anchored hearing system. For example, the data transfer antenna is configured to transmit a receiver data signal to an accessory device. An accessory device can be one or more of a smart phone, a tablet, and a computer.

The receiver data signal can be indicative of one or more operating parameters of the receiver. For example, the receiver data signal can be indicative of power level. The receiver data signal can be indicative of an error in the receiver. The receiver data signal can be indicative of temperature (which may be indicative of a fever). The receiver data can be indicative of a measured output level of a test signal for calibration and/or compensation of one or more components of the receiver. The receiver data signal can be indicative of skin thickness of the user.

In other words, the data transfer antenna can be configured to receiver and/or transmit data. For example, the receiver can be configured for bi-directional transmission (e.g., communication) with a transmitter of the bone-anchored hearing system.

In one or more example receivers, the data transfer antenna is located within the energy receiver coil. In other words, the data transfer antenna and the energy receiver coil are located along the same plane. For example, the energy receiver coil can surround the data transfer antenna. The data transfer antenna could be located within a central gap of the energy receiver coil. In other words, the data transfer antenna is nested within the energy receiver coil.

In one or more example receivers, the energy receiver coil is located within the data transfer antenna. In other words, the data transfer antenna and the energy receiver coil are located along the same plane. For example, the data transfer antenna can surround the energy receiver coil. The energy receiver coil could be located within a central gap of the data transfer antenna. In other words, the energy receiver coil is nested within the data transfer antenna.

In one or more example receivers, the data transfer antenna and the energy receiver coil are stacked. For example, the data transfer antenna and the energy receiver coil can be aligned with a magnetic field of the transmitter. In other words, the data transfer antenna and the energy receiver coil are not located along the same plane. The data transfer antenna may be closer to the user's skull, when the receiver is implanted, than the energy receiver coil. The energy receiver coil may be closer to the user's skull, when the receiver is implanted, than the data transfer antenna.

In one or more example receivers, the receiver further comprises a magnet. The magnet can be used to align the transmitter which can also include a transmitter magnet configured to attract to the magnet. In certain examples, the magnet can be nested within the energy receiver coil and/or the data transfer antenna.

In one or more example receivers, the energy signal and the data signal are provided in parallel. For example, the receiver circuit can be configured to provide the energy signal and the data signal in parallel. In other words, the circuitry within the receiver can include parallel circuitry for the energy signal and the data signal.

In one or more example receivers, the receiver comprises a single housing. In one or more example receiver, the signal housing contains the data transfer antenna, the energy receiver coil, the receiver circuit, and the vibrator transducer.

In certain examples, the receiver can comprise a first housing and a second housing. The first housing and the second housing can be attached. The first housing and the second housing can be attached via a connector. The connector can be a silicone connector. In one or more example receivers, the receiver comprises a first housing containing the data transfer antenna and the energy receiver coil and a second housing containing the receiver circuit and the vibrator transducer.

In one or more example receivers, the receiver is configured to modify the first resonant frequency and/or the second resonant frequency. This can allow the receiver to work at different resonant frequencies. In certain examples, the transmitted data signal can be indicative of a change in resonant frequency for the first resonant frequency and/or the second resonant frequency. In other words, the receiver is configured to tun the first resonant frequency and/or the second resonant frequency.

In one or more example receivers, the receiver circuit can be configured to perform the modifying. For example, the receiver circuit can include tuneable electronic components.

It may be advantageous to modify the first resonant frequency and/or the second resonant frequency if more or less energy was needed to operate the receiver. For example, skin thickness of the user may affect the energy needed.

The bone-anchored hearing system (e.g., a hearing aid) may be adapted to provide a frequency dependent gain and/or a level dependent compression and/or a transposition (with or without frequency compression) of one or more frequency ranges to one or more other frequency ranges, e.g. to compensate for a hearing impairment of a user. The hearing aid may comprise a signal processor for enhancing the input signals and providing a processed output signal.

The hearing aid may comprise an output unit for providing a stimulus perceived by the user as an acoustic signal based on a processed electric signal. The output unit may be a vibrator transducer of a bone conducting hearing aid. The output transducer may comprise a vibrator transducer for providing the stimulus as mechanical vibration of a skull bone to the user (e.g. in a bone-attached or bone-anchored hearing aid). The output unit may (additionally or alternatively) comprise a (e.g. wireless) transmitter for transmitting sound picked up-by the hearing aid to another device, e.g. a far-end communication partner (e.g. via a network, e.g. in a telephone mode of operation).

The hearing aid may comprise an input unit, such as on a transmitter, for providing an electric input signal representing sound. The input unit may comprise an input transducer, e.g. a microphone, for converting an input sound to an electric input signal. The input unit may comprise a wireless receiver for receiving a wireless signal comprising or representing sound and for providing an electric input signal representing said sound.

The wireless receiver and/or transmitter may e.g. be configured to receive and/or transmit an electromagnetic signal in the radio frequency range (3 kHz to 300 GHz). The wireless receiver and/or transmitter may e.g. be configured to receive and/or transmit an electromagnetic signal in a frequency range of light (e.g. infrared light 300 GHz to 430 THz, or visible light, e.g. 430 THz to 770 THz).

The hearing aid may comprise a directional microphone system adapted to spatially filter sounds from the environment, and thereby enhance a target acoustic source among a multitude of acoustic sources in the local environment of the user wearing the hearing aid. The directional system may be adapted to detect (such as adaptively detect) from which direction a particular part of the microphone signal originates. This can be achieved in various different ways as e.g. described in the prior art. In hearing aids, a microphone array beamformer is often used for spatially attenuating background noise sources. The beamformer may comprise a linear constraint minimum variance (LCMV) beamformer. Many beamformer variants can be found in literature. The minimum variance distortionless response (MVDR) beamformer is widely used in microphone array signal processing. Ideally the MVDR beamformer keeps the signals from the target direction (also referred to as the look direction) unchanged, while attenuating sound signals from other directions maximally. The generalized sidelobe canceller (GSC) structure is an equivalent representation of the MVDR beamformer offering computational and numerical advantages over a direct implementation in its original form.

Most sound signal sources (except the user's own voice) are located far away from the user compared to dimensions of the hearing aid, e.g. a distance d_{mic} between two microphones of a directional system. A typical microphone distance in a hearing aid is of the order 10 mm. A *minimum* distance of a sound source of interest to the user (e.g. sound from the user's mouth or sound from an audio delivery device) is of the order of 0.1 m (> 10 d_{mic}). For such minimum distances, the hearing aid (microphones) would be in the acoustic near-field of the sound source and a difference in level of the sound signals impinging on respective microphones may be significant. A *typical* distance for a communication partner is more than 1 m (>100 d_{mic}). The hearing aid (microphones) would be in the acoustic far-field of the sound source and a difference in level of the sound signals impinging on respective microphones is insignificant. The difference in *time of arrival* of sound impinging in the direction of the microphone axis (e.g. the front or back of a normal hearing aid) is ΔT= d_{mic}/v_{sound}=0.01/343 [s]=29 µs, where v_{sound} is the speed of sound in air at 20°C (343 m/s).

The hearing aid, such as the transmitter, may comprise antenna and transceiver circuitry allowing a wireless link to an entertainment device (e.g. a TV-set), a communication device (e.g. a telephone), a wireless microphone, a separate (external) processing device, or another hearing aid, etc. The hearing aid may thus be configured to wirelessly receive a direct electric input signal from another device. Likewise, the hearing aid may be configured to wirelessly transmit a direct electric output signal to another device. The direct electric input or output signal may represent or comprise an audio signal and/or a control signal and/or an information signal.

In general, a wireless link established by antenna and transceiver circuitry of the hearing aid can be of any type. The wireless link may be a link based on near-field communication, e.g. an inductive link based on an inductive coupling between antenna coils of transmitter and receiver parts. The wireless link may be based on far-field, electromagnetic radiation. Preferably, frequencies used to establish a communication link between the hearing aid and the other device is below 70 GHz, e.g. located in a range from 50 MHz to 70 GHz, e.g. above 300 MHz, e.g. in an ISM range above 300 MHz, e.g. in the 900 MHz range or in the 2.4 GHz range or in the 5.8 GHz range or in the 60 GHz range (ISM=Industrial, Scientific and Medical, such standardized ranges being e.g. defined by the International Telecommunication Union, ITU). The wireless link may be based on a standardized or proprietary technology. The wireless link may be based on Bluetooth technology (e.g. Bluetooth Low-Energy technology, e.g. LE audio), or Ultra WideBand (UWB) technology.

The hearing aid may comprise a 'forward' (or 'signal') path for processing an audio signal between an input and an output of the hearing aid. A signal processor may be located in the forward path. The signal processor may be adapted to provide a frequency dependent gain according to a user's particular needs (e.g. hearing impairment). The hearing aid may comprise an 'analysis' path comprising functional components for analyzing signals and/or controlling processing of the forward path. Some or all signal processing of the analysis path and/or the forward path may be conducted in the frequency domain, in which case the hearing aid comprises appropriate analysis and synthesis filter banks. Some or all signal processing of the analysis path and/or the forward path may be conducted in the time domain.

An analogue electric signal representing an acoustic signal may be converted to a digital audio signal in an analogue-to-digital (AD) conversion process, where the analogue signal is sampled with a predefined sampling frequency or rate fₛ, fₛ being e.g. in the range from 8 kHz to 48 kHz (adapted to the particular needs of the application) to provide digital samples xₙ (or x[n]) at discrete points in time tₙ (or n), each audio sample representing the value of the acoustic signal at tₙ by a predefined number N_{b} of bits, N_{b} being e.g. in the range from 1 to 48 bits, e.g. 24 bits. Each audio sample is hence quantized using N_{b} bits (resulting in 2^{Nb} different possible values of the audio sample). A digital sample x has a length in time of 1/fₛ, e.g. 50 µs, for *fₛ* = 20 kHz. A number of audio samples may be arranged in a time frame. A time frame may comprise 64 or 128 audio data samples. Other frame lengths may be used depending on the practical application.

The hearing aid may comprise an analogue-to-digital (AD) converter to digitize an analogue input (e.g. from an input transducer, such as a microphone) with a predefined sampling rate, e.g. 20 kHz. The hearing aids may comprise a digital-to-analogue (DA) converter to convert a digital signal to an analogue output signal, e.g. for being presented to a user via an output transducer.

The hearing aid, e.g. the input unit, and or the antenna and transceiver circuitry may comprise a transform unit for converting a time domain signal to a signal in the transform domain (e.g. frequency domain or Laplace domain, Z transform, wavelet transform, etc.). The transform unit may be constituted by or comprise a TF-conversion unit for providing a time-frequency representation of an input signal. The time-frequency representation may comprise an array or map of corresponding complex or real values of the signal in question in a particular time and frequency range. The TF conversion unit may comprise a filter bank for filtering a (time varying) input signal and providing a number of (time varying) output signals each comprising a distinct frequency range of the input signal. The TF conversion unit may comprise a Fourier transformation unit (e.g. a Discrete Fourier Transform (DFT) algorithm, or a Short Time Fourier Transform (STFT) algorithm, or similar) for converting a time variant input signal to a (time variant) signal in the (time-)frequency domain. The frequency range considered by the hearing aid from a minimum frequency fₘₘ to a maximum frequency fₘₐₓ may comprise a part of the typical human audible frequency range from 20 Hz to 20 kHz, e.g. a part of the range from 20 Hz to 12 kHz. Typically, a sample rate fₛ is larger than or equal to twice the maximum frequency fₘₐₓ, fₛ ≥ 2fₘₐₓ. A signal of the forward and/or analysis path of the hearing aid may be split into a number *NI* of frequency bands (e.g. of uniform width), where *NI* is e.g. larger than 5, such as larger than 10, such as larger than 50, such as larger than 100, such as larger than 500, at least some of which are processed individually. The hearing aid may be adapted to process a signal of the forward and/or analysis path in a number *NP* of different frequency channels (*NP* ≤ *NI*)*.* The frequency channels may be uniform or non-uniform in width (e.g. increasing in width with frequency), overlapping or non-overlapping.

The hearing aid may further comprise other relevant functionality for the application in question, e.g. compression, noise reduction, etc.

### A bone-anchored hearing system:

The above-disclosed receiver can be a part of a bone-anchored hearing system. For example, the bone-anchored hearing system can include a receiver such as discussed above. The bone-anchored hearing system can further include a transmitter. The transmitter can have at least one transmitter coil (e.g., at least one transmitter antenna). The transmitter can be configured to communicate with the data transfer antenna and/or the energy receiver coil. For example, transmitter can be configured to communicate with the data transfer antenna and/or the energy receiver coil via the at least one receiver coil.

The transmitter can be located on an outer skin surface of the hearing aid user. The transmitter can be positioned in order to transfer the energy signal and/or the data signal to the receiver. The transmitter can be attached to the user. For example, the transmitter can include a transmitter magnet that is attracted to a magnet in the receiver. This can help align the transmitter with the receiver. The receiver can be attached to the user via screws or adhesive.

The at least one transmitter coil can be configured to transmit the transmitted data signal at the first resonant frequency. The at least one transmitter coil can be configured to transmit the transmitted energy signal at the second resonant frequency. The at least one transmitter coil can be configured to change resonant frequencies for transmission.

The at least one transmitter coil can be a plurality of transmitter coils. In one or more example bone-anchored hearing systems, the at least one transmitter coil comprises a data transmitter coil and an energy transmitter coil. The data transmitter coil can transmit the transmitted data signal at the first resonant frequency. The energy transmitter coil can transmit the transmitted energy signal at the second resonant frequency. The data transmitter coil and the energy transmitter coil can be arranged in the transmitter in a similar manner to the data transfer antenna and the energy receiver coil of the receiver.

Advantageously, with the at least one transmitter coil being separate from the data transmitter coil, the specific electronic components around the respective coil can be optimized. For example, a high-Q value can be created for each of the resonant circuits.

In one or more example bone-anchored hearing systems, the bone-anchored hearing system further comprises an implantable battery. The receiver can be configured to charge the implantable battery with the energy signal. The implantable battery can be separate from the receiver. The implantable battery can be in electrical communication with the receiver. For example, the receiver can be configured to provide the energy signal to the implantable battery.

### Definitions:

In the present context, a hearing system, e.g., a hearing aid, e.g. a hearing instrument, refers to a device, which is adapted to improve, augment and/or protect the hearing capability of a user by receiving acoustic signals from the user's surroundings, generating corresponding audio signals, possibly modifying the audio signals and providing the possibly modified audio signals as audible signals to at least one of the user's ears. Such audible signals may e.g. be provided in the form of acoustic signals transferred as mechanical vibrations to the user's inner ears through the bone structure of the user's head and/or through parts of the middle ear.

The hearing aid may be configured to be worn in any known way, e.g. as a unit arranged behind the ear with a tube leading radiated acoustic signals into the ear canal or with an output transducer, e.g. a loudspeaker, arranged close to or in the ear canal, as a unit entirely or partly arranged in the pinna and/or in the ear canal, as a unit, e.g. a vibrator, attached to a fixture implanted into the skull bone, etc. The hearing aid may comprise a single unit or several units communicating (e.g. acoustically, electrically or optically) with each other. The loudspeaker may be arranged in a housing together with other components of the hearing aid, or may be an external unit in itself (possibly in combination with a flexible guiding element, e.g. a dome-like element).

A hearing aid may be adapted to a particular user's needs, e.g. a hearing impairment. A configurable signal processing circuit of the hearing aid may be adapted to apply a frequency and level dependent compressive amplification of an input signal. A customized frequency and level dependent gain (amplification or compression) may be determined in a fitting process by a fitting system based on a user's hearing data, e.g. an audiogram, using a fitting rationale (e.g. adapted to speech). The frequency and level dependent gain may e.g. be embodied in processing parameters, e.g. uploaded to the hearing aid via an interface to a programming device (fitting system), and used by a processing algorithm executed by the configurable signal processing circuit of the hearing aid.

A 'hearing system' can refer to a system comprising one or two hearing aids, and a 'binaural hearing system' refers to a system comprising two hearing aids and being adapted to cooperatively provide audible signals to both of the user's ears. Hearing systems or binaural hearing systems may further comprise one or more 'auxiliary devices', which communicate with the hearing aid(s) and affect and/or benefit from the function of the hearing aid(s). Such auxiliary devices may include at least one of a remote control, a remote microphone, an audio gateway device, an entertainment device, e.g. a music player, a wireless communication device, e.g. a mobile phone (such as a smartphone) or a tablet or another device, e.g. comprising a graphical interface. Hearing aids, hearing systems or binaural hearing systems may e.g. be used for compensating for a hearing-impaired person's loss of hearing capability, augmenting or protecting a normal-hearing person's hearing capability and/or conveying electronic audio signals to a person. Hearing aids or hearing systems may e.g. form part of or interact with public-address systems, active ear protection systems, handsfree telephone systems, car audio systems, entertainment (e.g. TV, music playing or karaoke) systems, teleconferencing systems, classroom amplification systems, etc.

The invention is set out in the appended set of claims.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
FIG. 1 illustrates an example embodiment of a receiver according to the disclosure,
FIG. 2 illustrates an example embodiment of a receiver according to the disclosure,
FIG. 3 illustrates an example embodiment of a receiver according to the disclosure,
FIG. 4 illustrates an example embodiment of a bone-anchored hearing system according to the disclosure, and
FIG. 5 illustrates an example embodiment of a bone-anchored hearing system according to the disclosure.

The figures are schematic and simplified for clarity, and they just show details which are essential to the understanding of the disclosure, while other details are left out. Throughout, the same reference signs are used for identical or corresponding parts.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description.

### DETAILED DESCRIPTION OF EMBODIMENTS

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include micro-electronic-mechanical systems (MEMS), integrated circuits (e.g. application specific), microprocessors, microcontrollers, digital signal processors (DSPs), field programmable gate arrays (FPGAs), programmable logic devices (PLDs), gated logic, discrete hardware circuits, printed circuit boards (PCB) (e.g. flexible PCBs), and other suitable hardware configured to perform the various functionality described throughout this disclosure, e.g. sensors, e.g. for sensing and/or registering physical properties of the environment, the device, the user, etc. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

The present application relates to the field of bone-anchored hearing systems, in particular receivers for use in bone-anchored hearing systems.

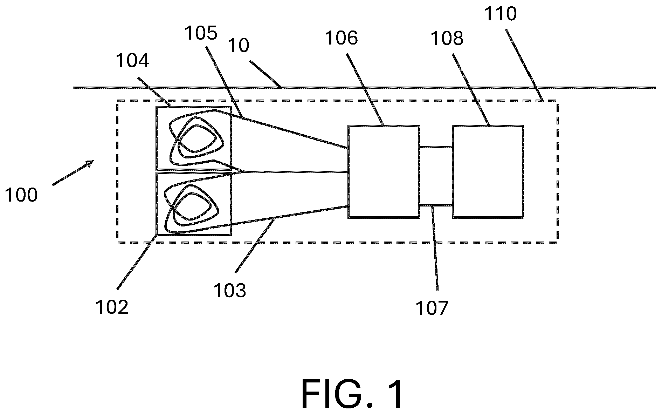

FIG. 1 illustrates an example embodiment of a receiver according to the disclosure. As shown, the receiver 100 is for a bone-anchored hearing system (BAHS). The receiver 100 can be attached (e.g., implanted) to the skull 10 of a hearing system user. The receiver 100 can be anchored to the skull 10 of the user. For example, the receiver 100 can be implanted to the skull 10 via screws, adhesives, etc. (not shown).

The receiver 100 includes a data transfer antenna 102 and an energy receiver coil 104. While shown in the particular position of FIG. 1, the position of the data transfer antenna 102 and energy receiver coil 104 can be reversed.

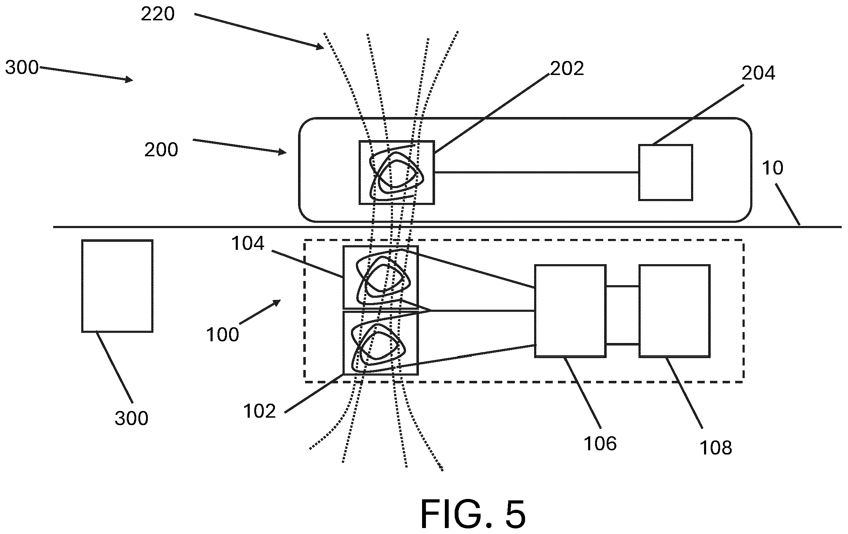

The data transfer antenna 102 is tuned at a first resonant frequency and configured to receive a transmitted data signal from a transmitter, wherein the data transfer antenna 102 is configured to provide a data signal 103 based on the transmitted data signal. The energy receiver coil 104 is tuned at a second resonant frequency and configured to receive a transmitted energy signal from the transmitter, wherein the energy receiver coil 104 is configured to provide an energy signal 105 based on the transmitted energy signal. The data transfer antenna 102 can be configured to transmit a receiver data signal, such as to a transmitter (shown in FIG. 5). The energy signal 105 and the data signal 103 cancan be provided in parallel, as shown.

The first resonant frequency may be different form the second resonant frequency of the same as the second resonant frequency, depending on the needs of the receiver. The receiver 100 is configured to modify the first resonant frequency and/or the second resonant frequency.

The receiver 100 further includes a receiver circuit 106. The receiver circuit 106 is configured to receive the energy signal 105 and the data signal 103, wherein the receiver circuit is configured to convert the data signal 103 to an output signal 107.

The receiver 100 includes a vibrator transducer 108 configured to receive the output signal 107 and to provide, based on the output signal 107, a vibration perceivable as sound by the user. Specifically, the vibrator transducer 108 applies a vibration to the skull 10 of the user.

The receiver 100 can include further components as well, such as electrical wiring, batteries, capacitors, amplifiers, etc. The receiver 100 can also include a housing 110 for containing the components of the receiver. For example, the receiver 100 can include a single housing 110 containing the data transfer antenna 102, the energy receiver coil 104, the receiver circuit 106, and the vibrator transducer 108.

As shown in FIG. 1, the data transfer antenna 102 and the energy receiver coil 104 can be stacked.

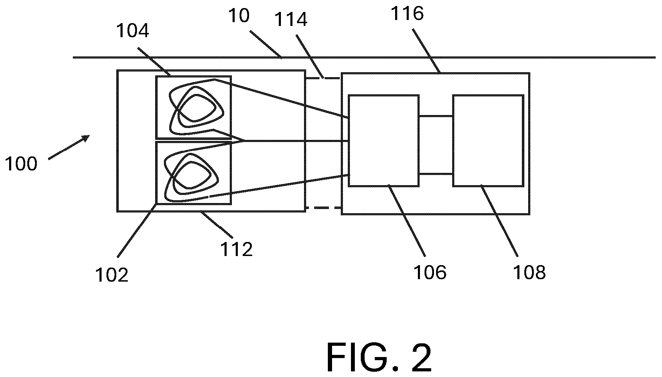

FIG. 2 illustrates an example embodiment of a receiver according to the disclosure. FIG. 2 incorporates any and/or all of the elements of FIG. 1. Unlike FIG. 1, the receiver of FIG. 2 includes a first housing 112 containing the data transfer antenna 102 and the energy receiver coil 104 as well as a second housing 116 containing the receiver circuit 106 and the vibrator transducer 108. The first housing 112 can be directly connected to the second housing 114. Optionally, the first housing 112 can be connected to the second housing 114 via a connector 106.

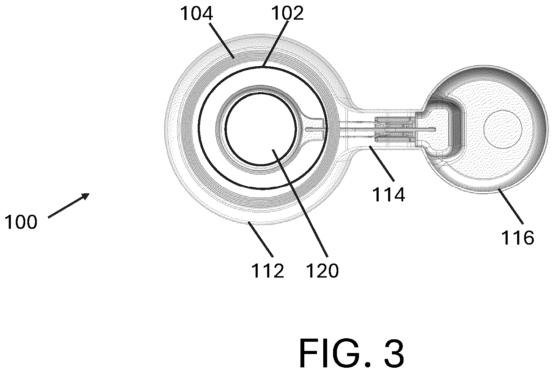

FIG. 3 illustrates an example embodiment of a receiver according to the disclosure. The receiver 100 can include any and/or all of the elements of FIGS. 1 and 2.

As shown in FIG. 3, the data transfer antenna 102 is located within the energy receiver coil 104. Alternatively, the energy receiver coil 104 is located within the data transfer antenna 102. Further, FIG. 3 illustrates that the receiver 100 can optionally include a magnet 120. FIG. 3 illustrates a two-housing embodiment, similar to the schematic of FIG. 2.

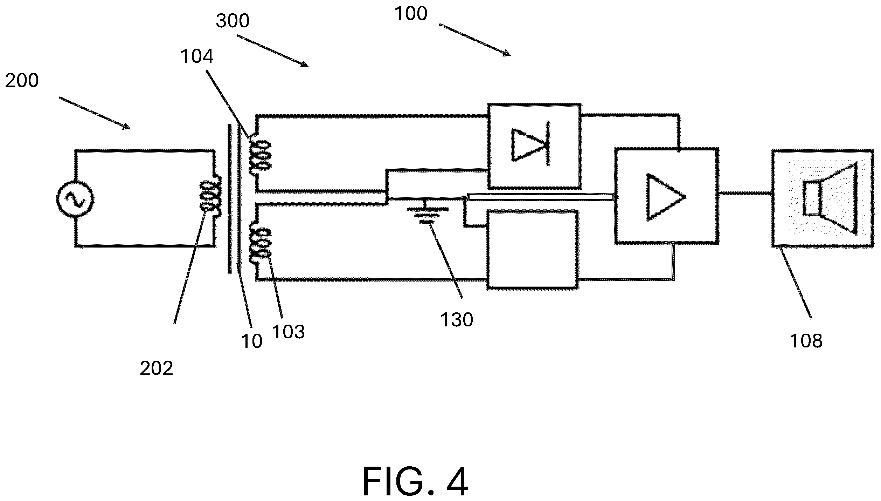

FIG. 4 illustrates an example embodiment of a bone-anchored hearing system according to the disclosure. As shown, the bone-anchored hearing system 300 can include a transmitter 200. The transmitter 200 can include at least one transmitter coil 202.

The bone-anchored hearing system 300 further includes the receiver 100. The receiver 100 can include an energy receiver coil 104 and a data receiver coil 103 (in certain examples, a data transfer antenna 102). The energy receiver coil 104 and the data receiver coil 103 can be connected at a point which is used as a common ground 130. The energy signal is rectified and used for driving the receiver electronics. The data signal is demodulated and the information then processed/amplified to drive the vibrator transducer 108.

FIG. 5 illustrates an example embodiment of a bone-anchored hearing system according to the disclosure. As shown, the bone-anchored hearing system 300 includes the receiver 100 such as disclosed herein. Further, the bone-anchored hearing system 300 can include a transmitter 200. The transmitter 200 can include at least one transmitter coil 202 and can be configured to communicate with the data transfer antenna 102 and the energy receiver coil 104. As shown, the transmitter 200, via the transmitter coil 202, can produce a magnetic field 220 that can be received by the data transfer antenna 102 and/or the energy receiver coil 104. The transmitter coil 202 can produce the magnetic field 220 in different resonant frequencies to be picked up by either the data transfer antenna 102 or the energy receiver coil 104.

The transmitter 100 can further include sound processing electronics 204 known for transmitters, such as processors, microphones (e.g., input transducers), etc.

While FIG. 5 illustrates the transmitter 200 with a single transmitter coil 202, alternatively the transmitter 200 can have an energy transmitter coil and a data transmitter coil. The two coils of the transmitter 200 can be tuned to the data transfer antenna 102 and the energy receiver coil 104, respectively.

The bone-anchored hearing system 300 can optionally include an implantable battery 300. The receiver 100 is configured to charge the implantable battery 300 with the energy signal.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art.

The claims are not intended to be limited to the aspects shown herein but are to be accorded the full scope consistent with the language of the claims, wherein reference to an element in the singular is not intended to mean "one and only one" unless specifically so stated, but rather "one or more." Unless specifically stated otherwise, the term "some" refers to one or more.

### ITEM LIST

Item 1. A receiver for a bone-anchored hearing system (BAHS), the receiver comprising:
a data transfer antenna tuned at a first resonant frequency and configured to receive a transmitted data signal from a transmitter, wherein the data transfer antenna is configured to provide a data signal based on the transmitted data signal;
an energy receiver coil tuned at a second resonant frequency and configured to receive a transmitted energy signal from the transmitter, wherein the energy receiver coil is configured to provide an energy signal based on the transmitted energy signal;
a receiver circuit configured to receive the energy signal and the data signal, wherein the receiver circuit is configured to convert the data signal to an output signal; and
a vibrator transducer configured to receive the output signal and to provide, based on the output signal, a vibration perceivable as sound by the user.

Item 2. The receiver of Item **1,** wherein the data transfer antenna is configured to transmit a receiver data signal.

Item 3. The receiver of any one of the previous Items, wherein the first resonant frequency is different from the second resonant frequency.

Item 4. The receiver of any one of Items 1-2, the first resonant frequency is the same as the second resonant frequency.

Item 5. The receiver of any one of the previous Items, wherein the data transfer antenna is located within the energy receiver coil.

Item 6. The receiver of any one of Items 1-4, wherein the energy receiver coil is located within the data transfer antenna.

Item 7. The receiver of any one of Items 1-4, wherein the data transfer antenna and the energy receiver coil are stacked.

Item 8. The receiver of any one of the previous Items, wherein the receiver is configured to be anchored to a skull of a user.

Item 9. The receiver of any one of the previous Items, wherein the receiver further comprises a magnet.

Item 10. The receiver of any one of the previous Items, wherein the energy signal and the data signal are provided in parallel.

Item 11. The receiver of any one of the previous Items, wherein the receiver comprises a single housing containing the data transfer antenna, the energy receiver coil, the receiver circuit, and the vibrator transducer.

Item 12. The receiver of any one of the previous Items, wherein the receiver comprises a first housing containing the data transfer antenna and the energy receiver coil and a second housing containing the receiver circuit and the vibrator.

Item 13. The receiver of any one of the previous Items, wherein the receiver is configured to modify the first resonant frequency and/or the second resonant frequency.

Item 14. A bone-anchored hearing system comprising:
a receiver according to any of the previous claims; and
a transmitter having at least one transmitter coil and configured to communicate with the data transfer antenna and the energy receiver coil.

Item 15. The bone-anchored hearing system of Item 14, wherein the at least one transmitter coil comprises a data transmitter coil and an energy transmitter coil.

Item 16. The bone-anchored hearing system of Item 14 or Item 15, wherein the bone-anchored hearing system further comprises an implantable battery, and wherein the receiver is configured to charge the implantable battery with the energy signal.

## Claims

1. A receiver for a bone-anchored hearing system (BAHS), the receiver comprising:
a data transfer antenna tuned at a first resonant frequency and configured to receive a transmitted data signal from a transmitter, wherein the data transfer antenna is configured to provide a data signal based on the transmitted data signal;
an energy receiver coil tuned at a second resonant frequency and configured to receive a transmitted energy signal from the transmitter, wherein the energy receiver coil is configured to provide an energy signal based on the transmitted energy signal;
a receiver circuit configured to receive the energy signal and the data signal, wherein the receiver circuit is configured to convert the data signal to an output signal; and
a vibrator transducer configured to receive the output signal and to provide, based on the output signal, a vibration perceivable as sound by the user.

2. The receiver of claim 1, wherein the data transfer antenna is configured to transmit a receiver data signal.

3. The receiver of any one of the previous claims, wherein the first resonant frequency is different from the second resonant frequency.

4. The receiver of any one of claims 1-2, the first resonant frequency is the same as the second resonant frequency

5. The receiver of any one of the previous claims, wherein the data transfer antenna is located within the energy receiver coil.

6. The receiver of any one of claims 1-4, wherein the energy receiver coil is located within the data transfer antenna.

7. The receiver of any one of claims 1-4, wherein the data transfer antenna and the energy receiver coil are stacked.

8. The receiver of any one of the previous claims, wherein the receiver is configured to be anchored to a skull of a user.

9. The receiver of any one of the previous claims, wherein the receiver further comprises a magnet.

10. The receiver of any one of the previous claims, wherein the receiver comprises a single housing containing the data transfer antenna, the energy receiver coil, the receiver circuit, and the vibrator transducer.

11. The receiver of any one of the previous claims, wherein the receiver comprises a first housing containing the data transfer antenna and the energy receiver coil and a second housing containing the receiver circuit and the vibrator transducer.

12. The receiver of any one of the previous claims, wherein the receiver is configured to modify the first resonant frequency and/or the second resonant frequency.

13. A bone-anchored hearing system comprising:
a receiver according to any of the previous claims; and
a transmitter having at least one transmitter coil and configured to communicate with the data transfer antenna and the energy receiver coil.

14. The bone-anchored hearing system of claim 13, wherein the at least one transmitter coil comprises a data transmitter coil and an energy transmitter coil.

15. The bone-anchored hearing system of claim 13 or claim 14, wherein the bone-anchored hearing system further comprises an implantable battery, and wherein the receiver is configured to charge the implantable battery with the energy signal.
